## Europäisches Patentamt
## European Patent Office
## Office européen des brevets

(19)

(11) Veröffentlichungsnummer: **0 146 144**
**B1**

(12) ## EUROPÄISCHE PATENTSCHRIFT

(45) Veröffentlichungstag der Patentschrift:
08.03.89

(51) Int. Cl.⁴: **A 61 K 39/085,** A 61 K 39/02,
A 61 K 41/00

(21) Anmeldenummer: 84115877.7

(22) Anmeldetag: 19.12.84

(54) Impfstoff und Verfahren zu seiner Herstellung.

(30) Priorität: 19.12.83 DE 3345864

(43) Veröffentlichungstag der Anmeldung:
26.06.85 Patentblatt 85/26

(45) Bekanntmachung des Hinweises auf die Patenterteilung:
08.03.89 Patentblatt 89/10

(84) Benannte Vertragsstaaten:
AT BE CH DE FR GB IT LI LU NL SE

(56) Entgegenhaltungen:
DE-C-539 018
GB-A-14 972

CHEMICAL ABSTRACTS, Band 97, Nr. 13, 27.
September 1982, Seite 491, Nr. 106647h, Columbus,
Ohio, US; D.J. LYNCH et al.: "Effects of potassium
sorbate on normal flora and on Staphylococcus
aureus added to minced cod"

(73) Patentinhaber: Freiherr von Maisen- Ponickau,
Egbert, P.O. Box/Appartado 428, Puerto de la
Cruz Teneriffa (ES)

(72) Erfinder: Freiherr von Maisen- Ponickau, Egbert,
P.O. Box/Appartado 428, Puerto de la Cruz
Teneriffa (ES)

(74) Vertreter: Glawe, Delfs, Moll & Partner
Patentanwälte, Rothenbaumchaussee 58
Postfach 2570, D-2000 Hamburg 13 (DE)

**Beschreibung**

Die Erfindung betrifft einen Impfstoff gegen durch pathogene Erreger verursachte Krankheiten bei Menschen oder warmblütigen Tieren.

Aufgabe der Erfindung ist es, für alle oder jedenfalls eine Reihe wichtiger durch pathogene Erreger, wie Viren, Bakterien und Pilze, erzeugte Krankheiten des Menschen oder der warmblütigen Tiere jeweils einen krankheitsspezifischen Impfstoff zu schaffen, der eine dauernde oder jedenfalls zeitweise Immunisierung gegen diese Krankheit und/oder eine Heilung bei bereits ausgebrochener Krankheit bewirkt.

Die Erfindung beruht auf der überraschenden Erkenntnis, daß es möglich ist, gezielt Formen von Erregern zu erzeugen, die ihre pathogenen Eigenschaften für Menschen oder warmblütige Tiere verloren haben, gleichwohl aber bei diesen Immunisierungs- und/oder Heilungswirkungen hervorrufen können.

Der erfindungsgemäße Impfstoff ist dadurch gekennzeichnet, daß er auf warmblüterfremdem tierischem Eiweiß lebensfähige Formen der betreffenden Erreger enthält. Vorzugsweise handelt es sich um Staphylococcen, insbesondere Staphylococcus aureus. Es können aber auch andere auf Fischeiweiß lebensfähige Formen von Bakterien und zwar insbesondere Streptococcen, Pneumococcen, Bacterium Pyocyaneum und Corinebakterien, sowie Salmonellen und andere pathogene Erreger verwendet werden. Außer Fischeiweiß kann auch Eiweiß von Crustaceen, Reptilien, Amphibien sowie auch Insekten und Würmern Verwendung finden.

Die Erfindung betrifft auch ein Verfahren zum Züchten von für Menschen oder warmblütige Tiere nicht pathogener Formen. Das Verfahren ist dadurch gekennzeichnet, daß man pathogene Erreger auf einen Nährboden mit nicht denaturiertem Eiweiß von nicht warmblütigen Tieren, insbesondere Fischeiweiß, impft und unter üblichen Züchtungsbedingungen eine Kolonie züchtet, wobei dieser Vorgang gegebenenfalls wiederholt wird. Vermutlich erfolgt dabei eine mutationsbedingte Anpassung, die die oben erwähnten Eigenschaften des fertigen Impfstoffes bewirkt.

Vorzugsweise läßt man gleichzeitig mutations- oder selektionsfördernde äußere Einflüsse, insbesondere Bestrahlung mit kurzwelliger elektromagnetischer Strahlung, Cobaltstrahlung und/oder Gammastrahlung, einwirken. Dabei hat sich insbesondere Ultraviolett-Strahlung als geeignet erwiesen.

Es ist besonders darauf hinzuweisen, daß mit dem gewonnenen Impfstoff pathogene Mikroorganismen bekämpft werden können.

Die Erfindung wird im folgenden durch Beispiele erläutert.

**Beispiel 1**

In einer Petrischale mit dem üblichen Nähragar wurde eine Staphylococcus aureus breitflächig ausgeimpft und im Brutschrank bei 37°C bebrütet.

Nach ausreichendem Wachstum wurde ein Teil dieser Bakterien mittels einer Platinnadel auf einen Nährboden in einer zweiten Petrischale ausgeimpft. Auch diese zweite Petrischale wurde im Brutschrank bei 37°C bebrütet, und außerdem der Strahlung einer UV-Lampe ausgesetzt.

Es wurde beobachtet, daß der größte Teil der Bakterien auf dem aus Fischeiweiß bestehenden Nährboden nicht lebensfähig war und abstarb. Nur wenige der ausgeimpften Bakterien überlebten.

Nach ausreichender Vermehrung wurde mittels einer Platinnadel eine erneute Überimpfung eines Teils der Bakterien auf einen dritten Nährboden vorgenommen. Man ließ nach der Bebrütung UV-Strahlung einwirken.

Dieser Vorgang wurde mit immer neuen Nährböden wiederholt.

Bei dem vorliegenden Beispiel waren über 150 Passagen erforderlich, was jedoch nicht ausschließt, daß bei der Reduplikation mehr oder weniger Passagen notwendig sind.

Aus den auf dem letzten Nährboden überlebenden Bakterien wurde unmittelbar eine Lebendvaccine hergestellt. Versuchstieren (Mäusen, Ratten, Meerschweinchen, Kaninchen) wurden Mengen dieses Impfstoffes eingespritzt, die bis zur vielfachen letalen Dosis des Ausgangsstammes entsprachen. Keines der geimpften Tiere starb oder zeigte Erkrankungserscheinungen. Anschließend wurden die geimpften Tiere sowie zur Kontrolle ungeimpfte mit einem entsprechenden virulenten Stamm infiziert. Auch bei Infektionsmengen, die ein Mehrfaches der bei den ungeimpften Tieren letalen Dosis betrugen, zeigten die geimpften Tiere sich völlig immun. Der Immunitätsgrad konnte beim Kaninchen durch Präzipitation nach Ouchtalony festgestellt werden.

**Beispiel 2**

In einer Petrischale mit einem Agar-Nährboden wie bei Beispiel 1 wurde eine Kolonie eines Staphylococcenstammes gezüchtet. Vermehrung und Überimpfung auf weitere Nährböden mit Fischeiweiß erfolgten wie bei Beispiel 1. Zur Förderung der Mutation wurde Bestrahlung mit langwelliger Röntgenstrahlung angewendet. Mit dem so behandelten Stamm wurde eine Lebendvaccine gewonnen.

Versuche gemäß Beispiel 1 und 2 wurden auch mit Streptococcen, insbesondere Streptococcus haemolyticus, sowie mit Bacterium pyocyanaeum und Corynebacterium diphteriae mit den gleichen Ergebnissen vorgenommen. Ferner wurde auch frisches, d.h. nicht denaturiertes Eiweiß von anderen, insbesondere von frisch getöteten oder frischen gefrorenen Fischen mit Erfolg angewendet. Das Eiweiß kann in Form von Suspensionen, Lösungen oder Dispersionen vorliegen. Unter geeigneter Anpassung der Züchtungsbedingungen, der Stärke der Bestrahlung dürften entsprechende nicht pathogene Mutationsformen von allen bisher bekannten oder ggf. noch zu entdeckenden pathogenen Bakterien herstellbar sein, sowie von Viren, Pilzen und Mykoplasmen. Besonders wichtig ist, daß auch bei bereits erkrankten Warmblütlern eine therapeutische Anwendung möglich ist.

**Patentansprüche**

1. Impfstoff gegen durch pathogene Erreger verursachte Krankheiten bei Menschen oder warmblütigen Tieren, dadurch gekennzeichnet, daß er auf warmblüterfremden tierischen Eiweiß lebensfähige Formen der betreffenden Erreger enthält.

2. Impfstoff nach Anspruch 1, dadurch gekennzeichnet, daß er auf Fischeiweiß lebensfähige Formen von Bakterien enthält.

3. Impfstoff nach Anspruch 1 oder 2, dadurch gekennzeichnet, daß als Erreger Staphylococcen, insbesondere Staphylococcus aureus, verwendet werden.

4. Verfahren zum Züchten von für Menschen oder warmblütige Tiere nicht pathogenen Formen pathogener Erreger, dadurch gekennzeichnet, daß man pathogene Erreger auf einen Nährboden mit Eiweiß von nicht warmblütigen Tieren, insbesondere Fischeiweiß impft und unter üblichen Züchtungsbedingungen eine Kolonie züchtet, wobei dieser Vorgang gegebenenfalls wiederholt wird.

5. Verfahren nach Anspruch 4, dadurch gekennzeichnet, daß man gleichzeitig mutations- bzw. selektionsfördernde Einflüsse, insbesondere Bestrahlung mit kurzwelliger elektromagnetischer Strahlung, einwirken läßt.

6. Verfahren nach Anspruch 5, dadurch gekennzeichnet, daß man nach ausreichender Besiedelung des letzten Nährbodens aus den lebenden Erregern ohne zusätzliche Verarbeitung unmittelbar eine Lebendvaccine herstellt.

**Claims**

1. Vaccine against diseases caused by pathogenic agents in humans or warm-blooded animals, characterized in that it contains forms of the respective agents capable of living on protein foreign to warm-blooded animals.

2. Vaccine according to claim 1, characterized in that it contains forms of bacteria capable of living on fish protein.

3. Vaccine according to claim 1 or 2, characterized in that as agents staphylococci, in particular Staphylococcus aureus are used.

4. Method of growing forms of pathogenic agents which are non-pathogenic for humans or warm-blooded animals, characterized in that pathogenic agents are inoculated into a culture medium with protein of non-warm-blooded animals, in particular fish protein and a colony grown under the usual cultivating conditions, this operation possibly being repeated.

5. Method according to claim 4, characterized in that at the same time mutation or selection-promoting influences are exerted, in particular irradiation with short-wave electromagnetic radiation.

6. Method according to claim 5, characterized in that after adequate colonization of the last culture medium a live vaccine is made directly from the living agents without additional processing.

**Revendications**

1. Inoculant vis-à-vis d'affections causées par des agents pathogènes chez l'homme ou chez les animaux à sang chaud, caractérisé en ce qu'il contient des formes viables sur de l'albumen d'animaux non à sang chaud de l'agent pathogène concerné.

2. Inoculant selon la revendication 1, caractérisé en ce qu'il contient des formes viables de bactéries sur de l'albumen de poisson.

3. Inoculant selon la revendication 1 ou 2, caractérisé en ce que des staphylocoques, notamment le staphylocoque doré, sont utilisés comme agents pathogènes.

4. Procédé pour la culture d'agents pathogènes sous des formes non pathogènes pour l'homme ou pour des animaux à sang chaud caractérisé en ce qu'on en cultive une colonie selon les conditions habituelles de culture, ce processus étant répété le cas échéant.

5. Procédé selon la revendication 4, caractérisé en ce qu'on fait intervenir simultanément des effets favorisant la mutation ou la sélection, notamment une exposition à un rayonnement électro-magnétique à ondes courtes.

6. Procédé selon la revendication 5, caractérisé en ce que, après une colonisation suffisante du dernier bouillon de culture, on fabrique directement un vaccin vivant, à partir des agents pathogènes vivants et sans traitement complémentaire.